# EUROPÄISCHE PATENTANMELDUNG

(11) **EP 0 904 752 A1**
(43) Veröffentlichungstag der Anmeldung: **31.03.1999**
(21) Anmeldenummer: 97116947.9
(22) Anmeldetag: 30.09.1997
(51) Int. Cl.: A61F 5/37

(54) **Bandagen-System**

(71) Anmelder: Habermeyer, Peter, Prof. Dr. med., 69115 Heidelberg (DE)
(72) Erfinder: Habermeyer, Peter, Prof. Dr. med., 69115 Heidelberg (DE)
(74) Vertreter: Richter, Werdermann & Gerbaulet

(57) **Zusammenfassung**

Um neben einer axialen Gewichtsentlastung der Schulter bei Oberarmfrakturen und Schulterluxationen gleichzeitig auch eine gesicherte Stellung des Oberarms in einer Abduktion zu erreichen, besteht ein Bandagensystem (100') zur axialen Gewichtsentlastung der Schulter bei Oberarmfrakturen und Schulterluxationen aus einer GILCHRIST-Bandage (50') und einem mit dieser fest oder lösbar verbundenen, den Oberarm in einer Abduktion von vorzugsweise 20° bis 30° haltenden, körperformgerecht ausgebildeten und sich oberhalb des Beckens abstützenden Abduktionskissen (150').

## Beschreibung

Die Erfindung betrifft ein Bandagen-System zur Anwendung bei Oberarmfrakturen und Schulterluxationen.

Fixierverbände für Oberarmfrakturen und Schulterluxationen sind in verschiedenen Ausführungsformen bekannt.

So ist aus der US-A-4 469 095 eine elastische, schlauchförmige, druckausübende Armbandage bekannt, deren schulterseitiges Ende mit einem schlingenförmig ausgebildeten Befestigungsband versehen ist, das mit einem Büstenhalterträgerband der jeweiligen Trägerin der Bandage verbunden werden kann, um ein Herabrutschen des Schlauchteiles zu verhindern. Eine derartige Kompressions-Bandage soll die Lymphdrainage unterstützen; sie ist jedoch nicht zur Fixierung eines verletzten Armes geeignet.

Dem DE-Prospekt "SCHUMACHER AKTUELL Tubigrip" der Firma F. & W. Schumacher KG, 86/1000 Druck: Papier Obermann, Krefeld und dem DE-Prospekt "SCHUMACHER AKTUELL Tubigrip" F. & W. Schumacher KG, (seit 1905) ist ein Gilchrist-Verband zu entnehmen, der aus einem Schlauchteil aus "Tubigrip" (Handelsmarke) hergestellt ist, einem Material, das elastisch dehnbar ist. Diese Art von Verbänden dient der Fixierung von Oberarmfrakturen und Schulterluxationen. Zur Herstellung dieses Fixierverbandes wird von dem Schlauchteil ausgegangen, der dem Mehrfachen einer Armlänge entspricht.

An der Grenze vom ersten zum zweiten Drittel des Schlauchteiles wird eine Kante des Schlauches in einer bestimmten Länge durch Einschneiden geschlitzt, wobei das lange Ende des Schlauches von hier aus über den kranken Arm gezogen wird. Das kurze Schlauchende wird über den Nacken gelegt und nach vorn geführt; es wird dann um das Handgelenk des angewinkelten Armes geschlungen und mit Sicherheitsnadeln befestigt. Das lange Schlauchende wird von den Fingerspitzen aus hinten um den Rumpf geführt, von innen um den kranken Oberarm oberhalb des Ellenbogengelenks geschlungen und ebenfalls mit Sicherheitsnadeln festgesteckt. Am Handgelenk kann der Schlauch zur Freigabe von Hand und Fingern durch Einschneiden mit einer Schere geschlitzt werden. Die Herstellung dieses Fixier-Verbandes aus einem Schlauchteil ist mühevoll; sie bedarf immer der Hilfe einer zweiten Person; ein selbsttätiges Anlegen des Verbandes ohne Hilfe einer weiteren Person durch den Patienten selbst ist nicht möglich, denn schon die Vorarbeiten an dem Schlauchteil sind aufwendig und nur von geübten Kräften durchführbar, denn eine unrichtige Längenmessung und ein falsches Einschneiden des Schlauches führt zu einem nicht mehr verwendbarer Verband. Diejenigen Abschnitte des Schlauchteiles, die dem Tragen bzw. der Halterung des Fixier-Verbandes dienen, werden aus dem Schlauchteil gebildet; sie lassen sich nicht in flach gelegtem Zustand einwandfrei um die Schulter und den Nacken legen; da es sich bei dem Material des Schlauches um ein elastisches Material handelt, neigt dieses leicht dazu, sich wulstartig zusammenzurollen, so daß es zu unerwünschten Einschnürungen und Strangulationen kommen kann, da insbesondere bei einer Schulter- oder Nackenbewegung des Patienten die Anfangs flach gelegten Schlauchabschnitte nicht ihre Lage beibehalten.

Ein Fixier-Verband für Oberarmfrakturen und Schulterluxationen unter Verwendung eines Unterarm und Oberarm aufnehmenden, aus einem radial elastischen, in Längsrichtung jedoch im wesentlichen undehnbaren Gewebematerial bestehenden Schlauchteil mit einem Trageband und einem Halteband und mit Verschlußorganen zur Bildung von Halteschlaufen ist aus der EP-A-0 198 482 bekannt. Bei diesem Verband ist an den Schlauchteilenden jeweils ein Manschettenbund aus nichtdehnbarem Material vorgesehen, an denen das Trageband und das Halteband befestigt ist, wobei das Trageband und das Halteband als Flachformbänder mit integrierter Versteifung ausgebildet sind. Die Verschlußorgane sind als Klettverschlußelemente ausgebildet.

Die bekannten Fixierbandagen sind ausschließlich bei Oberarmfraktionen und Schulterluxationen, jedoch nicht bei akromioklavikularen Luxationen einsetzbar, wobei es sich um eine Zerrung, eine Subluxation oder um eine totale Luxation handeln kann, wobei bei einer derartigen Acromioclaviculargelenk (AC)- Sprengung eine Behandlung dahingehend vorgenommen wird, daß auf das Schlüsselbeinende ein Druck ausgeübt werden muß, damit dem Schlüsselbein eine Bewegung in Richtung zum Acromion erteilt wird, um eine Heilung bzw. ein Zusammenwachsen der Bänder und Gelenkkapseln zu erreichen.

Durch direktes Trauma, z.B. durch einen Sturz vom Fahrrad, kommt es häufig zu Zerreißungen der Gelenkbänder und als Folge davon zu einem schmerzhaften Höherstehen des lateralen Schlüsselbeinendes. Das Großteil derartiger Verletzungen könnte konservativ behandelt werden, wenn es möglich wäre, das Schlüsselbein mittels einer Haltevorrichtung nach unten zu drücken und in seine gelenkige Verbindung zu reponieren und in dieser Position dauerhaft zu halten. Dieses Höhertreten des Schlüsselbeins bei gleichzeitigem Vermeiden eines nach unten gerichteten Zuges am Schulterblatt soll mit dem Bandagen-System nach der EP-A- 0 425 938 verhindert werden. Da aber dieses Bandagen-System aus einer Kreuzgurtanordnung besteht, bei der die Kreuzungsstelle der Gurte im Bereich des Schlüsselbeines gelegen ist, ergibt sich insbesondere dadurch, daß die wechselseitig einander zugeordneten freien Enden der Gurte paarweise einerseits im Bereich des distalen Oberarms und andererseits im Bereich des Handteils des abgewickelten, vor dem Oberkörper fixierten Unterarms befestigt sind, leicht ein Verrutschen der Gurte im Kreuzungsbereich, das noch bei einer Armbewegung gefördert wird, so daß das Höhertreten des Schlüsselbeins nicht immer verhindert werden kann mit der Folge, daß der Heilungsprozeß oftmals unterbrochen wird.

Das Bandagen-System gemäß der DE 43 14 758, insbesondere für akromioklavikulare Luxationen und laterale Claviculafrakturen, besteht aus einem Unterarm und Oberarm aufnehmenden, aus einem radial-elastischen, in Längsrichtung jedoch im wesentlichen undehnbaren Gewebematerial bestehenden Schlauchteil mit einem mit dem schulterseitigen Ende des Schlauchteils verbundenen Trageband, einem mit dem handseitigen Ende des Schlauchteils verbundenen Halteband und mit an den freien Enden von Trageband und Halteband vorgesehenen Verschlußorganen zur Bildung von Halteschlaufen und einem im angelegten Zustand des Verbandes ringförmigen, sich über die Schulter einerseits und um den angewinkelten Unterarm andererseits erstreckenden Zuggurt mit einem vorderseitigen Abschnitt als Brustgurt und einem rückwärtigen Abschnitt als Rückengurt besteht, wobei der Zuggurt in seiner Länge veränderbar ausgebildet ist. Hiernach ist die bekannte Fixier-Bandage nach Gilchrist mit einem zweckmäßigerweise über das äußere Drittel des Schlüsselbeines geführten Zuggurt ausgestaltet, der als Endlosband bzw. ringförmig ausgebildet ist und den Unterarm des Armes der verletzten Schulter aufnimmt. Mit einem derartigen Bandagen-System wird das Schlüsselbein gezielt niedergehalten, wobei aber bei einer ungewollten Armbewegung eine Verschiebung des Zuggurtes nicht auszuschließen ist, auch wenn der Zuggurt im Bereich des schlüsselbeinseitigen Endes des Schlauchteils des Bandagen-Systems lagegesichert gehalten ist, wobei eine Lagesicherung noch dadurch unterstützt werden kann, daß im Verbindungsbereich des Zuggurtes mit dem Schlauchteil das Trageband angreift, so daß durch die vom Trageband ausgeübten Zugkräfte zur Lagesicherung des Kreuzgurtes beitragen.

Ein Verschieben des Zuggurtes im Unterarmbereich ist nicht vermeidbar, da hier der Zuggurt mit seinem Ende schlaufenförmig um den abgewinkelten Unterarm herumgelegt ist. Im Bereich des Unterarms erfolgt keine Fixierung des Zuggurtes. Diese Verschiebbarkeit des Zuggurtes im Unterarmbereich kann dazu führen, daß ein Zug am Schulterblatt nach unten auftreten kann mit der Folge, daß ein Höhertreten der Clavicula nicht vermieden werden kann.

Ferner ist durch die DE-U- 94 16 590.4 ein Bandagen-System für akromioklavikulare Luxationen und laterale Claviculafrakturen bekannt, das aus einem Unterarm- und Oberarm aufnehmenden Schlauchteil mit einen mit dem schulterseitigen Ende des Schlauchteils verbundenen Trageband für den Unterarm, aus einem mit dem handseitigen Ende des Schlauchteils verbundenen Halteband mit am Oberarm gehaltenen freien Ende, wobei an den freien Enden von Trageband und Halteband Verschlußorgane vorgesehen sind, und aus einem im angelegtem Zustand der Bandage ringförmigen, sich über die Schulter einerseits und um den angewinkelten Unterarm andererseits erstreckenden Zuggurt mit einem vorderseitigen Abschnitt als Brustgurt und einem rückwärtigen Abschnitt als Rückengurt bekannt, wobei das Schlauchteil außenseitig an seinem im Bereich des Unterarms liegenden Abschnitt eine am Schlauchteil positionsveränderbare Führungslasche zur Aufnahme des Zuggurtes aufweist. Mit einem derart ausgebildeten anlagefertigen Bandagen-System ist die Behandlung von Schultergelenksprengungen und von Schlüsselbeinbrüchen möglich, wobei nicht nur durch entsprechende Druck- und Zugeinwirkung das Acromion und das Schlüsselbein so gegeneinandergedrückt werden, das ein Abheben des Schlüsselbeins von dem Acromion vermieden wird, sondern der Zuggurt am Unterarm in einer vorgegebenen und den jeweiligen Erfordernissen anpaßbaren Stellung fixierbar ist, so daß sichergestellt wird, das einerseits kein Zug am Schulterblatt nach unten und andererseits kein Höhertreten der Clavicula entstehen kann. Allerdings ist mit diesem Bandagen-System und allen anderen bekannten Bandagen eine Lagerung des Armes in Abduktionsstellung nicht möglich.

Ferner ist es bekannt, bei Rupturen der Rotaturenmanschette, beim Ausriß des Sehnenansatzes oder bei der Ruptur der Supraspinatussehne zur Ruhigstellung der Schulter und des Armes neben abnehmbaren Schulterabduktionsverbänden, Abduktionsschienen einzusetzen, die oftmals so ausgebildet sind, daß sie für die rechte und linke Körperseite verwendet werden können. Derartige Abduktionsschienen werden z.B. aus einem Drahtgestell hergestellt und am Körper mittels elastischer Binden angewinkelt.

Aufgabe der vorliegenden Erfindung ist es ein Bandagen-System zu schaffen, mit dem neben einer axialen Gewichtsentlastung der Schulter bei Oberarmfrakturen und Schulterluxationen auch eine gesicherte Stellung des Oberarms in einer Abduktion möglich ist.

Gelöst wird diese Aufgabe bei einem Bandagen-System mit den in den Ansprüchen 1, 2, 3 und 4 angegebenen Merkmalen.

Hiernach besteht die Erfindung in der Einbindung eines Abduktionskissens in eine Gurtanordnung entsprechend GILCHRIST, wobei diese Gurtanordnung zur axialen Gewichtsentlastung der Schulter bei Oberarmfrakturen und Schulterluxationen eingesetzt wird. Diese Gurtanordnung besteht aus einem Unter- und Oberarm aufnehmenden Schlauchteil mit einem mit dem schulterseitigen Ende des Schlauchteils verbundenen, um den Nacken des Patienten geführten Trageband für den Unterarm und aus einem um den Körper herumgeführten Halte- bzw. Körperband bzw. aus einem mit dem handseitigen Ende des Schlauchteils verbundenen Halteband mit an dem Oberarm gehaltenen freien Ende, wobei an den freien Enden von dem Trageband und dem Halteband Verschlußorgane vorgesehen sind.

In diese Gurtanordnung ist das Abduktionskissen eingebunden, das den Oberarm in eine Abduktion von 20° bis 30° hält und Körperform gerecht ausgebildet ist, so daß das Abduktionskissen im angelegten Zustand sich oberhalb des Beckens abstützt. Das Abduktionskissen besteht aus einem Schaumstoff oder einem anderen geeigneten Material mit einem vorderen breiten Teil und einem sich nach hinten verjüngenden Teil, und ist vorderseitig mit dem Halteband und mit dem Trageband lösbar verbunden, wobei vor dem Anschluß des Haltebandes an den Oberarm das Halteband bevorzugterweise durch eine Schlaufe am rückwärtigen Teil des Abduktionskissens geführt ist, um das Abduktionskissen zu fixieren und in der durch die Gurtanordnung vorgegebenen Stellung zu halten, wobei auch eine Halterung des Abduktionskissens in seinem rückwärtigen Bereich vermittels eines Klettverschlusses erfolgen kann.

Eine weitere Ausführungsform der Erfindung sieht ein Bandagen-System vor, das zur axialen Gewichtsentlastung der Schulter bei Oberarmfrakturen und Schulterluxationen aus einer Gurtanordnung aus einem Unterarm und Oberarm aufnehmenden Schlauchteil mit einem mit dem schulterseitigen Ende des Schlauchteils verbundenen, um den Nacken geführten Trageband und mit einer elastischen Daumenschlinge an dem freien Ende des den Unterarm aufnehmenden Abschnittes des Schlauchteils, und aus einem den Oberarm in einer Abduktion von vorzugsweise 20° bis 30° haltenden, körperformgerechten, sich im angelegten Zustand oberhalb des Beckens am Patientenkörper abstützenden Abduktionskissen aus einem Schaumstoff oder einem anderen geeigneten Material mit einem rückwärtigen breiteren, der Körperform angepaßten Teil und einem schmal nach vorn auslaufenden Teil, wobei die Länge des Abduktionskissens in etwa der Länge des Unterarms des Patienten entspricht und das Abduktionskissen an seiner Außenwandfläche zur Halterung des an der Außenwandfläche anliegenden Unterarms mindestens eine Unterarmhalteschlinge aufweist, und aus einem mit dem rückwärtigen Ende des Abduktionskissen und mit dessen vorderen Ende lösbar verbundenen, um den Patientenkörper herumgelegten Körperband besteht, wobei das Abduktionskissen im vorderen Endbereich mit Anschlußmitteln für die lösbare Befestigung des Tragebandes versehen ist.

Um den Oberarm und den Unterarm an dem Abduktionskissen Lage-fixiert zu halten, weist das Abduktionskissen im Anlagebereich des Unterarmes eine erste muldenförmige Ausnehmung, die in Abduktionskissenlängsrichtung verläuft, und im Anlagebereich des Oberarmes eine quer zur Abduktionskissenlängsrichtung verlaufende zweite muldenförmige Ausnehmung auf.

Mit dem erfindungsgemäß ausgebildeten Bandagen-System wird eine gesicherte Rotationsstellung des Armes erreicht. Hierzu trägt die beim Bandagen-System eingesetzte Gurtanordnung wesentlich bei, so daß die Erfindung in der Kombination einer Gurt-Anordnung mit einem Abduktionskissen besteht, durch das der Arm in einer Abduktionsstellung gehalten wird. Der Unterarm kann sich auf der Oberfläche des Abduktionskissens abstützen jedoch besteht auch die Möglichkeit den Unterarm seitlich an dem Abduktionskissen zu halten.

Eine weitere Ausgestaltung der Erfindung sieht die Anordnung des Abduktionskissens am Schlauchteil selbst und zwar dessen Unterarmbereich vor.

Eine lösbare Befestigungsmöglichkeit zwischen dem Schlauchteil und dem Abduktionskissen besteht darin, daß das Schlauchteil im Unterarmbereich mittels eines Klettverschlusses an der Außenwandfläche bzw. Seitenwandfläche des Abduktionskissens oder auf der Oberseite des Abduktionskissens befestigt und gehalten wird.

Weitere vorteilhafte Ausgestaltung der Erfindung gehen aus den Unteransprüchen hervor.

Ausführungsbeispiele der Erfindung werden nachfolgend anhand der Zeichnungen näher erläutert. Es zeigen
- Fig. 1: eine Vorderansicht des Bandagen-Systems in angelegtem Zustand mit einem den Arm in eine leichte Abduktionsstellung haltenden Abduktionskissen, wobei der Unterarm seitlich an dem Abduktionskissen anliegt,
- Fig. 2: eine Vorderansicht des Bandagen-Systems im angelegten Zustand mit einem den Arm in eine leichte Abduktionsstellung haltenden Abduktionskissen, wobei der Unterarm auf dem Abduktionskissen aufliegt,
- Fig. 3: in einer schaubildlichen Ansicht ein an der Gurtanordnung befestigtes Abduktionskissen,
- Fig. 4: eine schaubildliche Ansicht einer weiteren Ausführungsform des Abduktionskissens mit einer oberen Aufnahmemulde für den Unterarm,
- Fig. 5: eine schaubildliche Ansicht eines Abduktionskissens mit einer vorderseitig angeformten, keilartigen Abstützfläche für die Hand,
- Fig. 6: in einer schaubildlichen Ansicht einen GILCHRIST-Bandagenteil aus einem Unterarm und Oberarm aufnehmenden Schlauchteil und einem mit dem schulterseitigen Ende des Schlauchteils verbundenen, um den Nacken herumführbaren Trageband und mit einer am freien handseitigen Ende des Schlauchteils vorgegebener Daumenschlinge,
- Fig. 7: in einer schaubildlichen Ansicht den mit einem Abduktionskissen kombinierten GILCHRIST-Bandagenteil gemäß Fig. 6,
- Fig. 8: in einer schaubildlichen Ansicht das Abduktionskissen gemäß Fig. 7 mit einem Körpergurt,
- Fig. 9: in einer weiteren schaubildlichen Ansicht das Abduktionsnkissen gemäß Fig. 8, und
- Fig. 10: eine Ansicht in Pfeilrichtung A in Fig. 9 auf das Abduktionskissen.

Das erfingungsgemäße Bandagen-System 100, 100' besteht aus der Kombination einer in an sich bekannter Weise ausgebildeten GILCHRIST-Bandage bzw. GILCHRIST-Bandagenteil und einem Abduktionskissen 150, 150' in den verschiedensten Ausführungsformen, das fest oder lösbar mit der GILCHRIST-Bandage oder dem GILCHRIST-Bandagenteil verbunden ist.

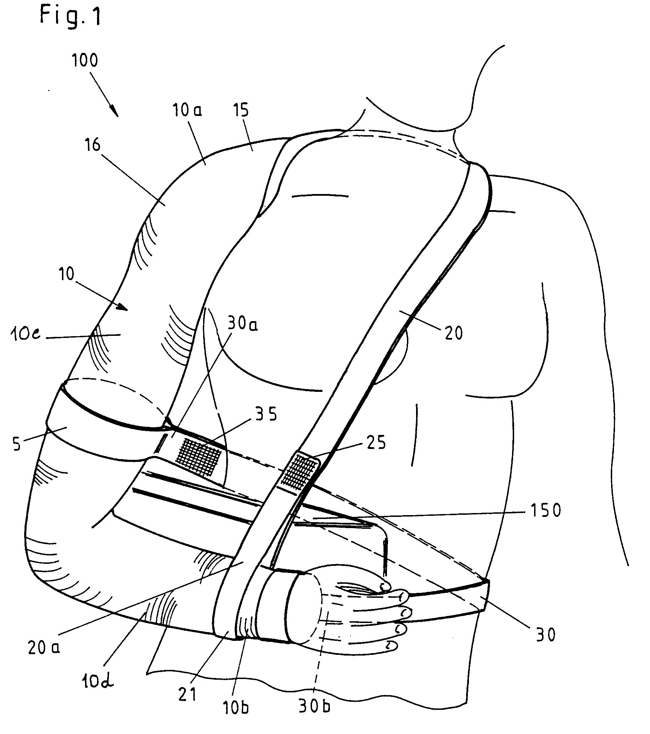

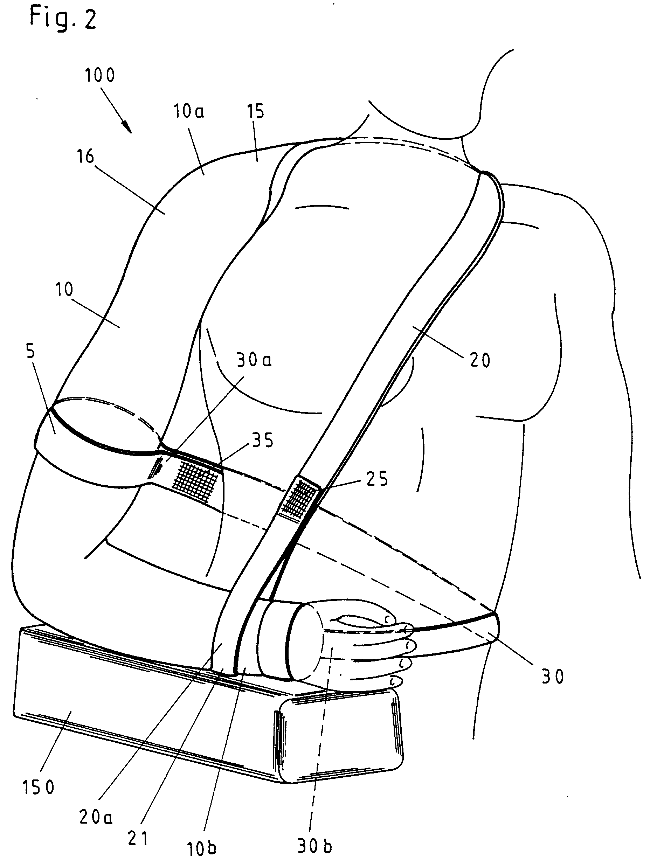

Das in den Fig. 1 und 2 dargestellte Bandagen-System 100 besteht aus einem Unter- und Oberarm aufnehmenden Schlauchteil 10 mit dem schulterseitigen Ende 10a und dem handseitigen Ende 10b. Das Schlauchteil 10 besteht aus einem radial elastischen, zweckmäßigerweise in Längsrichtung, jedoch im wesentlichen undehnbaren Gewebematerial. Die Länge des Schlauchteils 10 entspricht in etwa der Länge eines Armes. Der den Unterarm aufnehmende Schlauchabschnitt ist mit 10d und der den Oberarm aufnehmende Schlauchabschnitt mit 10e bezeichnet.

An das schulterseitige Ende 10a des Schlauchteils 10 ist ein Trageband 20 und an das handseitige Ende 10b des Schlauchteils 10 ist eine Halteband 30 angeschlossen, wobei das Trageband 20 und das Halteband 30 aus einem Schlauchabschnitt bei der Herstellung des Schlauchteiles 10 mit herausgeschnitten werden können. Bevorzugterweise sind jedoch das Trageband 20 und das Halteband 30 selbständige Bandagenteile, die mittels Nähverbindungen an den beiden Enden 10a, 10b des Schlauchteils befestigt sind. Beide Bänder 20, 30 sind aus gepolstertem Gurtmaterial gefertigt.

Das Ende 20a des Tragebandes 20 ist mit einem Verschlußorgan 25 zur Bildung einer Halteschlaufe 21 versehen. Dieses Verschlußorgan 25 ist bevorzugterweise als links und rechts verwendbare Klettverschlüsse ausgebildet. Durch das dem Oberarm zugekehrte Ende 30a ist schlaufenförmig um den Oberarm herumgelegt. Und zur Bildung der Schlaufe 31 ist das freie Bandende mit einem Verschlußorgan 35 versehen, das z.B. aus einem Klettverschluß gebildet ist. Mittels des Tragebandes 20 ist somit eine Schlaufe 21 um den Unterarm und mittels des Haltebandes 30 eine Schlaufe 31 um den unteren Bereich des Oberarmes des Patienten gelegt. Durch die Schlaufeneinstellung über die Klettverschlüsse kann die Kraft, mittels der der Arm gegen den Oberkörper des Patienten gedrückt wird, praktisch stufenlos eingestellt werden. Anstelle eines Klettverschlusses können auch andersartig ausgebildete und geeignete Verschlußorgane eingesetzt werden.

Das mit seinem einen Ende am handseitigen Ende 10b des Schlauchteils 10 an diesem befestigte Halteband 30 weist nach einer weiteren Ausführungsform eine Länge auf, die in etwa der doppelten Breite des Rückens eines Patienten entspricht, so daß das Halteband 30 nach Umschlingung des Oberarms bei 31 an der Rückenseite zurückgeführt und mit seinem freien Ende mittels einer Klettverschlußverbindung an dem darunterliegenden Abschnitt des Haltebandes 30 befestigt werden kann. Dadurch, daß die Endbefestigung des Haltebandes 30 in etwa im seitlichen Körperbereich erfolgt, trägt das Halteband 30 im Rückenbereich nicht auf und es kann somit auch zu keinen Druckstellen kommen.

Das Trageband 20 und das Halteband 30 sind als Flachformbänder, insbesondere mit integrierter Versteifung, ausgebildet. Um diese Flachform des Tragebandes 20 und des Haltebandes 30 im angelegten Zustand des Bandagen-Systems 100 beizubehalten, können alle drei Bänder ein- und mehrlagig ausgebildet sein, wobei vermittels Steppnähten und evtl. integrierter Polsterung erreicht wird, daß die beiden Bänder 20, 30 im angelegten Zustand des Bandagen-Systems ihre Flachform beibehalten.

Um das Schlauchteil 10 im angelegten Zustand des Bandagen-Systems 100 möglichst weit in den Schulterbereich zu ziehen, geht die armaußenseitige Wandfläche des Schlauchteils 10 im Bereich seines schulterseitigen Endes 10a in einen etwa ellipsenförmigen Gewebezuschnitt 15 zur Ausbildung einer Schulterkappe 16 über, an bzw. auf der das Trageband 20 befestigt ist. Das Trageband 20 und das Halteband 30 bestehen zweckmäßigerweise aus einem undehnbaren Gewebematerial.

Es besteht jedoch auch die Möglichkeit, das Trageband 20 und das Halteband 30 aus einem undehnbaren Gewebematerial auszubilden.

Das schulterseitige Schlauchteilende 10a bzw. der den Gewebezuschnitt 15 begrenzende Rand kann ebenso wie das handseitige Ende 10b des Schlauchteils 10 mit einem Bündchen aus einem nichtdehnbarem Material versehen sein, wobei dann das Trageband 20 an dem Bündchen am schulterseitigen Ende 10a bzw. auf dem Gewebezuschnitt 15 des Schlauchteils 10 befestigt ist.

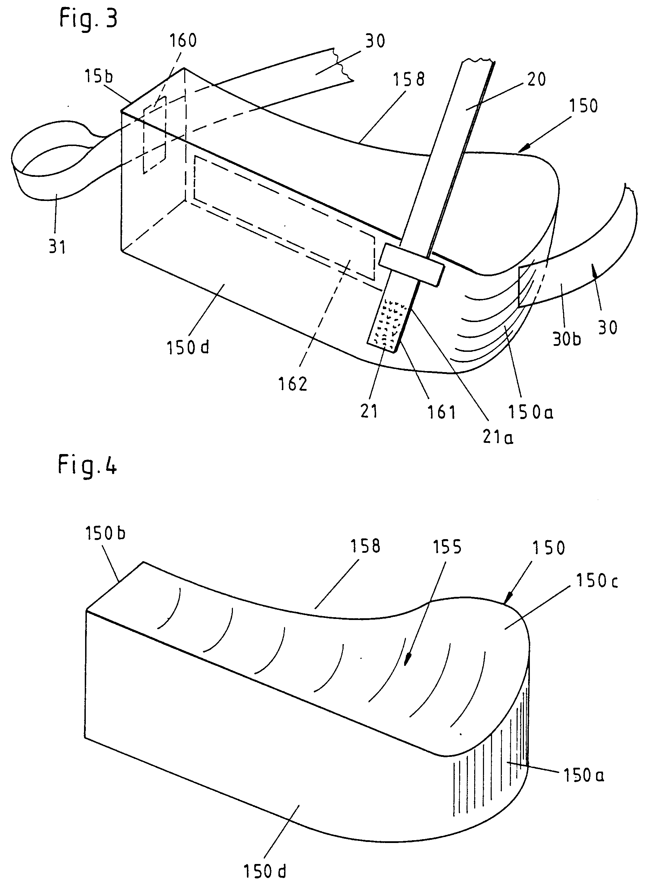

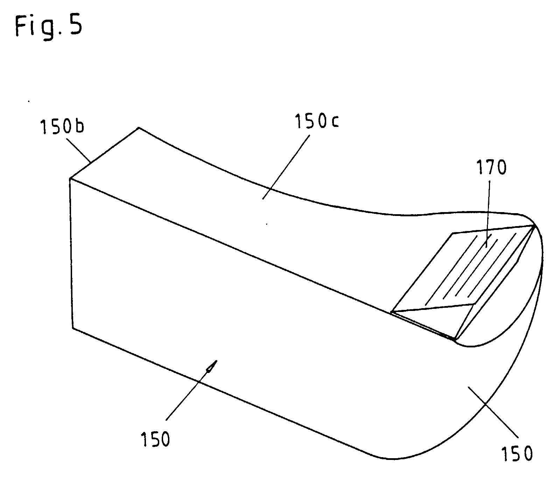

Um den Arm in einer Abduktionsstellung zu halten, ist die aus dem Schlauchteil 10, dem Trageband 20 und dem Halteband 30 bestehende Gurtanordnung 50 mit einem Abduktionskissen 150 verbunden, das in die Gurtanordnung 50 integriert ist und das von den Bändern 20, 30 so gehalten ist, daß in Verbindung mit der Ausgestaltung des Abduktionskissens 150 der Oberarm in einer Abduktion von vorzugsweise 20° bis 30° gehalten wird.(Fig. 1 und 2) das Abduktionskissen 150 besteht aus einem etwa quaderförmigen Formkörper aus einem Schaumkunststoff oder einen anderen geeigneten Material, das polsterartige Eigenschaften besitzt, wobei das Kissen 150 eine körpergerechte Form aufweist, d.h. im seitlich angelegtem Zustand stützt sich das Kissen 150 oberhalb des Beckens am Körper des Patienten ab. Danach weist das Abduktionskissen 150 einen vorderen breiten Teil 150a und einen sich zum rückwärtigen Bereich hin verjüngenden Teil 150b auf, wobei im körperauflageseitigen Bereich das Abduktionskissen 150 eine der Körperform angepaßte Ausnehmung 158 auf (Fig. 3 und 4).

Das Abduktionskissen 150 kann in die Gurtanordnung 20, 30 so eingebunden sein, daß der Unterarm seitlich an dem Abduktionskissen 150 anliegt (Fig.1) oder auf dem Abduktionskissen aufliegt (Fig. 2).

Die Halterung des Abduktionskissens 150 erfolgt bei einer seitlichen Anlage des Unterarms an dem Abduktionskissen in der Weise, daß das oberarmseitige Ende 30a des Haltebandes 30 durch eine Schlaufe 160 am rückwärtigen Teil 150b des Abduktionskissen 150 hindurchgeführt wird, woraufhin dann die den Oberarm umschlingende Schlaufe 31 gebildet wird. Das handseitige Ende 30b des Haltebandes 30 ist am vorderen Teil 150a des Abduktionskissens befestigt (Fig. 3). Es besteht auch noch die Möglichkeit, zusätzlich das unterarmseitige Ende 20a des Tragebandes 20 vor der Ausbildung der Schlaufe 21 zur Aufnahme des Unterarms durch eine Schlaufe 161 an der äußeren Seitenwand 150d des Abduktionskissens 150 hindurchzuführen (Fig. 3). Anstelle der Schlaufen 21, 31 können auch Klettverschlußverbindungen vorgesehen sein.

Eine weitere zusätzliche seitliche Halterung des Unterarms an dem Abduktionskissen 150 kann in der Weise erfolgen, daß das Schlauchteil 10 im Unterarmbereich und zwar innenseitig Klettverschlußteile aufweist, die in entsprechende Gegenklettverschlußteile eingreifen, die in Fig. 3 bei 162 angedeutet sind. Das Abduktionskissen 150 wird somit mittels der Bänder 20, 30 und des Schlauchteils 10 in Stellung seitlich am Körper gehalten. Die Abduktion des Oberarmes sollte dabei bei einer Winkelstellung von 20° bis 30° liegen, jedoch sind andere Winkelstellungen durchaus möglich.

Durch Veränderung der Längen und Sitz der Bänder 20, 30 kann das Abduktionskissen 150 in jede gewünschte Stellung gebracht werden.

Nach einer weiteren Ausführungsform gemäß Fig. 2 erfolgt die Abstützung des Unterarmes auf der Oberfläche 150c des Abduktionskissens 150, das eine in Kissenlängsrichtung verlaufende muldenförmige Vertiefung 155, in die der Unterarm eingelegt und Lage gesichert ist (Fig. 4).

Zur Handauflage kann gemäß Fig. 5 das Abduktionskissen 150 in seinem vorderen Teil 150a mit einer keilförmigen Anformung 170 versehen sein.

Die Einbindung des Abduktionskissen 150 in die Gurtanordnung aus den beiden Bänder 20, 30 ist nicht beschränkt auf die vorangehend beschriebene Ausführungsform. Neben einer festen Anordnung der Bänder 20, 30 an dem Abduktionskissen 150 kann auch eine lösbare Verbindung vorgesehen sein.

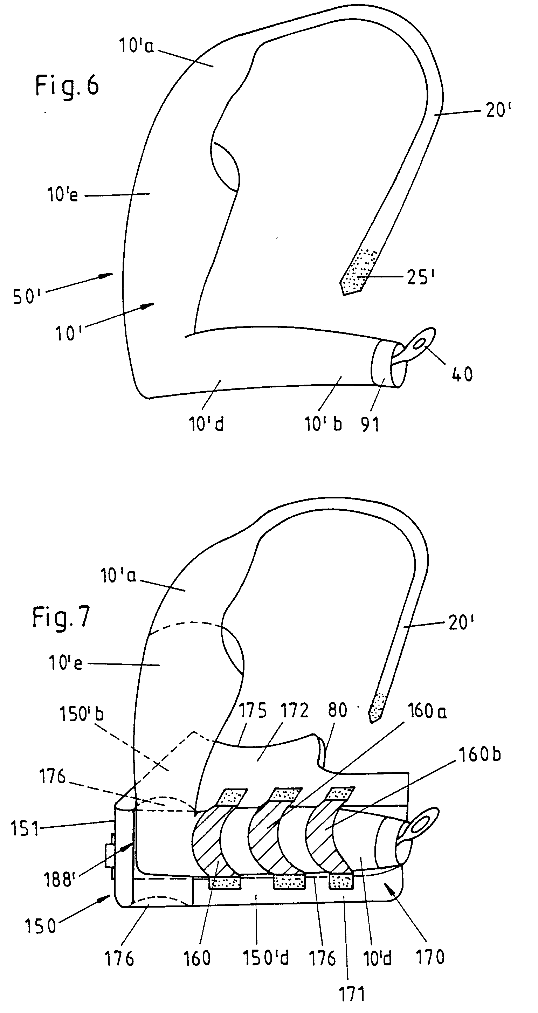

Eine weitere Ausführungsform eines Bandagen-Systems 100' zur axialen Gewichtsentlastung der Schulter bei Oberarmfrakturen und Schulterluxationen ist in den Fig. 6 bis 10 dargestellt.

Dieses Bandagen-System 100' besteht gemäß Fig. 6 aus einem Unterarm und Oberarm aufnehmenden Schlauchteil 10' mit einem mit dem Schulterseiten Ende 10'a des Schlauchteils 10' verbundenen, um den Nacken geführten Trageband 20' und mit einer elastischen Daumenschlinge 40 an dem freien Ende 10'b des den Unterarm aufnehmenden Abschnittes des Schlauchteiles 10'.

Des weiteren besteht das Bandagen-System 100' aus einem den Oberarm in einer Abduktion von vorzugsweise 20° bis 30° haltenden, körperformgerecht ausgebildeten und sich im angelegten Zustand oberhalb des Beckens am Patientenkörper abstützenden Abduktionskissen 150', welches fest oder lösbar mit der Gurtanordnung 50' verbunden ist (Fig. 7). Das Abduktionskissen 150' besteht entsprechend dem Abduktionskissen 150 aus einem Schaumstoff oder einem anderen geeigneten Material.

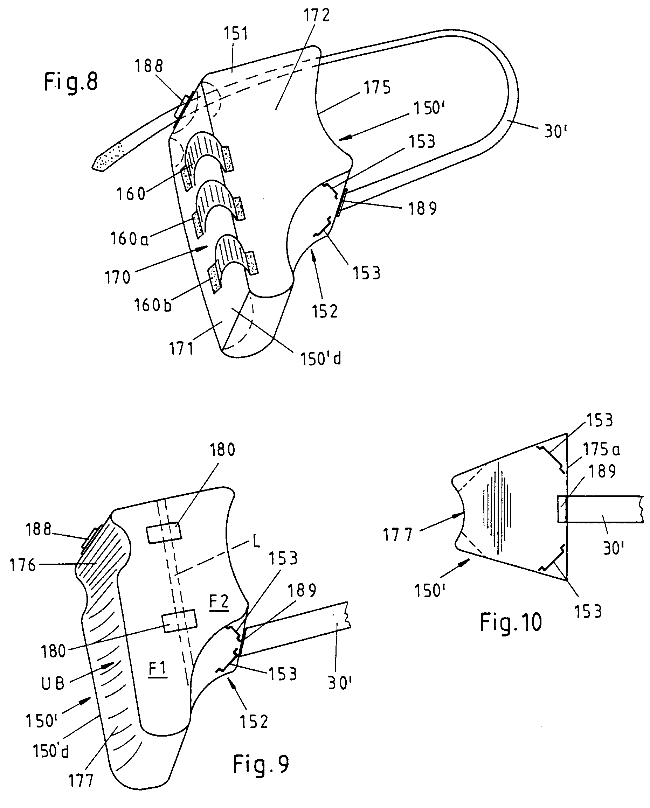

Um eine gute Körperanlage zu erreichen, ist das Abduktionskissen 150' mit einer breiten, seitlichen Anlagefläche versehen. Die Formgestaltung des Abduktionskissens 150' entspricht der in den Fig. 7, 8 und 9 dargestellten Ausführungsform. Danach besteht das Abduktionskissen 150' aus einem etwa L-förmigen Formkörper 170, dessen längere, in etwa der Länge des Unterarmes entsprechende Schenkel 171 an seiner Außenwandfläche 150'd Halteschlaufen 160, 160a, 160b für den Unterarm trägt, wobei der kürzere Schenkel 172 des Formkörpers 170 an seinem den längeren Schenkel 171 abgekehrten Ende mit einer der Körperform entsprechend ausgebildeten Ausnehmung 175 versehen ist. Danach weist das Abduktionskissen 150' einen rückwärtigen breiteren, der Körperform angepaßten Teil 150'b und einen schmal nach vorn auslaufenden Teil 150'a auf, wobei die Länge des Abduktionskissens 150' in etwa der Länge des Unterarmes des Patienten entspricht. An seiner Außenwandfläche 150'd weist das Abduktionskissen 150' zur Halterung des an der Außenwandfläche 150'd anliegenden Unterarms mindestens eine Unterarmhalteschlinge 160 auf; bei dem in den Fig. 7 und 8 gezeigten Ausführungsbeispiel sind bevorzugterweise drei Unterarmhalteschlingen 160, 160a, 160b vorgesehen. Diese Unterarmhalteschlingen 160, 160a, 160b, die schlaufenförmig ausgebildet sind, sind an der Außenwandfläche 150'd des Abduktionskissens 150' einendseitig an der Außenwandfläche 150'd befestigt und mit ihrem anderen Ende lösbar an der Außenwandfläche 150'd gehalten. Bevorzugterweise bestehen die Unterarmhalteschlingen aus VELCRO-Bändern. Diese Unterarmhalteschlingen 160, 160a, 160b sind

einendseitig, d.h. mit ihrem freien Ende an der Außenwandfläche 150'd des Abduktionskissens 150' mittels eines Klettverschlusses gehalten, wobei auch andere Befestigungsmittel vorgesehen sein können, wie z.B. Schnallen oder Druckknopfverschlüsse. Es besteht auch die Möglichkeit, diese Unterarmhalteschlingen bzw. -schlaufen längenveränderbar auszubilden, um eine Anpassung an die Stärke des Unterarms des Patienten vornehmen zu können.

Des weiteren umfaßt das Bandagen-System 100' ein mit dem rückwärtigen Ende 151 des Abduktionskissens 150' vermittels einer Schlaufe, Schnalle oder einer Klettverschlußverbindung 188 verbundenes, um den Patientenkörper herumgelegtes Körperband 30' auf, das mit dem vorderen Ende 152 vermittels einer Schlaufe, Schnalle oder einer Klettverschlußverbindung 189 lösbar mit dem Abduktionskissen 150' verbunden ist. Das Abduktionskissen 150' ist im vorderen Endbereich 152 mit Anschlußmitteln 153 für die lösbare Befestigung des Tragebandes 20' des Schlauchteiles 10' der Gurtanordnung 50' versehen.

Das Schlauchteil 10' mit dem Trageband 20' in Verbindung mit dem Körperband 30' bildet im Prinzip eine GILCHRIST-Bandage bzw. -Bandagenteil entsprechend der Gurtanordnung 50.

Wie Fig. 6 zu entnehmen ist, ist das Trageband 20' des Schlauchteils 10' endseitig als Verschlußmittel 25' ausgebildet, z.B. in Form eines zu einer Schlaufe ausbildbaren Endabschnittes, der an dem Abduktionskissen 150' an einem Steg, Schlaufe o.dgl. 80 befestigbar ist (Fig. 7).

Das freie, handseitige Ende des den Unterarm aufnehmenden Schlauchabschnittes 10'd ist mit einem verstärkt ausgebildeten, d.h. stabilen Handgelenksbund 91 versehen, an den die elastische Daumenschlinge 40 befestigt ist. Der den Oberarm aufnehmende Schlauchabschnitt des Schlauchteiles 10' ist mit 10'e bezeichnet (Fig. 6).

Wie Fig. 10 zeigt weist das Abduktionskissen 150' eine breite Körperanlagefläche 175a auf.

Für die Befestigung sowohl des Tragebandes 20' als auch des Körperbandes 30' können die verschiedensten Anschlußmittel eingesetzt und verwendet werden, so u.a. Klettverschlußverbindungen, Schnallenverbindungen oder Druckknopfverbindungen. Außerdem kann das Trageband 20' und das Körperband 30' längenveränderbar ausgebildet sein. Beide Bänder 20', 30' sind entsprechend den Bändern 20, 30 des Bandagen-Systemes 100 ausgebildet und bestehen aus den gleichen Materialien.

Zur besseren Fixierung und Lagenstabilisierung des Unterarms und des Oberarms des Patienten an dem Abduktionskissen 150', weist dieses im Oberarmanlagebereich OB eine im Querschnitt bogenförmig verlaufende und der Form des Oberarms angepaßten muldenförmigen Ausnehmung 176 auf (Fig. 9).

Darüber hinaus ist das Abduktionskissen 150' an seiner Außenwandfläche 150'd im Unterarmanlagebereich UB mit einer in Abduktionskissenlängsrichtung verlaufenden im Querschnitt bogenförmig ausgebildeten muldenförmigen Ausnehmung 177 zur Unterarmfixierung versehen (Fig. 9).

Das Abduktionskissen 150' kann entweder die muldenförmige Ausnehmung 176 zur Lagenfixierung des Oberarms oder die muldenförmige Ausnehmung 177 zur Unterarmfixierung aufweisen, jedoch wird das Abduktionskissen 150' bevorzugterweise mit beiden muldenförmigen Ausnehmungen 176, 177 versehen sein.

Nach einer weiteren Ausführungsform gemäß Fig. 9 ist das Abduktionskissen 150' in Längsrichtung L verlaufend zweigeteilt ausgebildet und besteht somit aus zwei Formkörperteilen F1, F2, die über oben- und untenseitig verlaufende elastische Bänder 180 miteinander verbunden sind, wobei in den zwischen den beiden Formteilen F1, F2 ausgebildeten Zwischenraum abstandsveränderbare Zwischenstücke aus dem Material des Abduktionskissens 150' zur Änderung des Abduktionswinkels einführbar sind. Je nach Wahl der Breite der Zwischenstücke ist der jeweils gewünschte Abduktionswinkel erreichbar. Anstelle von Zwischenstücken aus formstabilen Material kann auch ein Zwischenstück in Form eines mittels eines gasförmigen Mediums füllbaren Formkörper verwendet werden, wobei dieser Formkörper auch im gefüllten Zustand eine Querschnittsform aufweist, die in etwa der Querschnittsform der beiden Formkörperteile F1, F2 im Bereich ihrer Trennlinie aufweist. Derartige Formkörper können auch mit einem flüssigen Medium gefüllt werden. Dies hat den Vorteil, daß der Abduktionswinkel mühelos und quasi stufenlos verändert werden kann.

Das Abduktionskissen 150 des Bandagen-Systems 100 kann in gleicher Weise wie das Abduktionskissen 150' zweigeteilt ausgebildet sein, d.h. aus zwei Formkörper bestehen.

## Patentansprüche

1. Bandagen-System,
dadurch gekennzeichnet,
daß das Bandagen-System (100; 100') zur axialen Gewichtsentlastung der Schulter bei Oberarmfrakturen und Schulterluxationen aus einer GILCHRIST-Bandage (50; 50') und einem mit dieser fest oder lösbar verbundenen, den Oberarm in einer Abduktion von vorzugsweise 20° bis 30° haltenden, körperformgerecht ausgebildeten und sich oberhalb des Beckens abstützenden Abduktionskissen (150; 150') besteht.

2. Bandagen-System,
dadurch gekennzeichnet,
daß das Bandagen-System (100; 100') zur axialen Gewichtsentlastung der Schulter bei Oberarmfrakturen und Schulterluxationen
a) aus einer GILCHRIST-Bandage (50; 50') aus einem Unterarm und Oberarm aufnehmenden Schlauchteil (10; 10'), aus einem mit dem schulterseitigen Ende (10a; 10'a) des Schlauchteils (10; 10') verbundenen, um den Nacken geführten Trageband (20; 20') und aus einem um den Körper herumgeführten Halte- bzw. Körperband (30; 30'), und
b) aus einem den Oberarm in einer Abduktion von vorzugsweise 20° bis 30° haltenden, körperformgerechten, sich im angelegten Zustand oberhalb des Beckens des Patienten abstützenden Abduktionskissen (150; 150') aus einem Schaumstoff oder einem anderen geeigneten Werkstoff besteht, wobei das Trageband (20; 20') mit seinem freien Ende an einem Ende des Abduktionskissen (150; 150') und das Halte- bzw. Körperband (30; 30') an den beiden Enden des Abduktionskissens (150; 150') fest oder lösbar verbunden ist und wobei der Unterarm auf dem Abduktionskissen (150; 150') oder an seiner seitlichen Außenwand gehalten ist.

3. Bandagen-System,
dadurch gekennzeichnet,
daß das Bandagen-System (100) zur axialen Gewichtsentlastung der Schulter bei Oberarmfrakturen und Schulterluxationen
a) aus einer Gurtanordnung (50) aus einem Unter- und Oberarm aufnehmenden Schlauchteil (10) mit einem mit dem schulterseitigen Ende (10a) des Schlauchteils (10) verbundenen, um den Nacken geführten Trageband (20) für den Unterarm und aus einem mit dem handseitigen Ende (10b) des Schlauchteils (10) verbundenen Halteband (30) mit am Oberarm gehaltenen freien Ende (30a), wobei an den freien Enden (20a, 30a) von dem Trageband (20) und dem Halteband (30) Verschlußorgane (25, 35) vorgesehen sind, und
b) aus einem dem Oberarm in einer Abduktion von vorzugsweise 20° bis 30° haltenden, körperformgerechten, sich im angelegten Zustand oberhalb des Beckens am Patientenkörper abstützenden Abduktionskissen (150) aus einem Schaumstoff oder einem anderen geeigneten Material mit einem vorderen breiten Teil (150a) und einem sich nach hinten verjüngenden Teil (150b) besteht, wobei das Abduktionskissen (150) vorderseitig mit dem Halteband (30) und mit dem Trageband (20) lösbar verbunden ist.

4. Bandagen-System,
dadurch gekennzeichnet,
daß das Bandagen-System (100') zur axialen Gewichtsentlastung der Schulter bei Oberarmfrakturen und Schulterluxationen
a) aus einer Gurtanordnung (50') aus einem Unterarm und Oberarm aufnehmenden Schlauchteil (10') mit einem mit dem schulterseiten Ende (10'a) des Schlauchteils (10') verbundenen, um den Nacken geführten Trageband (20') und mit einer elastischen Daumenschlinge (40) an dem freien Ende (10'b) des den Unterarm aufnehmenden Abschnittes des Schlauchteils (10') ,
und
b) aus einem den Oberarm in einer Abduktion von vorzugsweise 20° bis 30° haltenden, körperformgerechten, sich im angelegten Zustand oberhalb des Beckens am Patientenkörper abstützenden Abduktionskissen (150') aus einem Schaumstoff oder einem anderen geeigneten Material mit einem rückwärtigen breiteren, der Körperform angepaßten Teil (150'b) und einem schmal nach vorn auslaufenden Teil (150'a), wobei die Länge des Abduktionskissens (150') in etwa der Länge des Unterarms des Patienten entspricht und das Abduktionskissen (150') an seiner Außenwandfläche (150'd) zur Halterung des an der Außenwandfläche (150'd) anliegenden Unterarms mindestens eine Unterarmhalteschlinge (160) aufweist, und
c) aus einem mit dem rückwärtigen Ende (151) des Abduktionskissens (150') vermittels einer Schlaufe, Schnalle oder einer Klettverschlußverbindung (188) und mit dessen vorderen Ende (152) vermittels einer Schlaufe, Schnalle oder einer Klettverschlußverbindung (189) lösbar verbundenen, um den Patientenkörper herumgelegten Körperband (30') besteht, wobei das Abduktionskissen (150') im vorderen Endbereich (152) mit Anschlußmitteln (153) für die lösbare Befestigung des Tragebandes (20') versehen ist.

5. Bandagen-System nach einem der Ansprüche 1 bis 3,
dadurch gekennzeichnet,
daß das oberarmseitige Ende (30a) des Haltebandes (30) vor der Befestigung am Oberarm durch eine Schlaufe (160) am hinteren Teil (150b) des Abduktionskissens (150) geführt ist.

6. Bandagen-System nach einem der Ansprüche 1 bis 5,
dadurch gekennzeichnet,
daß der Arm mittels der Gurtanordnung (50), in einer seitlichen Anlage an dem Abduktionskissen (150) gehalten wird.

7. Bandagen-System nach einem der Ansprüche 1 bis 6,
dadurch gekennzeichnet,
daß das Schlauchteil (10) im Unterarmbereich mittels einer Klettverbindung (162) an der Außenwand (150d) des Abduktionskissens (150) gehalten ist.

8. Bandagen-System nach einem der Ansprüche 1 bis 3, 5 bis 7,
dadurch gekennzeichnet,
daß in der Oberfläche (150c) des Abduktionskissen (150) eine in Kissenlängsrichtung verlaufende muldenförmige Vertiefung (155) zur Aufnahme des Unterarms ausgebildet ist, wobei der Oberarm und der Unterarm in der muldenartigen Vertiefung (155) in dem Abduktionskissen (150) mittels der Gurtanordnung (50) gehalten wird.

9. Bandagen-System nach einem der Ansprüche 1 bis 3, 5 bis 8,
dadurch gekennzeichnet,
daß das Trageband (20) mit seinem handseitigen Ende (20a) durch eine Schlaufe (161) an der Außenwandfläche (150d) des Abduktionskissens (150) vor der Befestigung am Unterarm geführt ist.

10. Bandagen-System nach einem der Ansprüche 1 bis 3, 5 bis 9,
dadurch gekennzeichnet,
daß die Schlaufen (160, 161) als Klettverschlußverbindungen ausgebildet sind.

11. Bandagen-System nach einem der Ansprüche 1 bis 3, 5 bis 10,
dadurch gekennzeichnet,
daß das Abduktionskissen (150) in seinem vorderen Teil (150a) auf seiner oberen Wandfläche (150c) eine keilförmige Anformung (170) zur Handauflage und -abstützung aufweist.

12. Bandagen-System nach Anspruch 4,
dadurch gekennzeichnet,
daß das Abduktionskissen (150') an seiner längsverlaufenden Außenwandfläche (150'd) drei den Unterarm haltende, bevorzugterweise längenveränderbare Unterarmhalteschlingen (160, 160a, 160b) aufweist.

13. Bandagen-System nach einem der Ansprüche 4 und 12,
dadurch gekennzeichnet,
daß das Abduktionskissen (150') aus einem etwa L-förmigen Formkörper (170) besteht, dessen längeren, in etwa der Länge des Unterarms entsprechende Schenkel (171) an seiner Außenwandfläche (150'd) die Halteschlaufen (160, 160a, 160b) für den Unterarm trägt, wobei der kürzere Schenkel (172) des Formkörpers (170) an seinem den längeren Schenkel (171) abgekehrten Ende mit einer der Körperform entsprechend ausgebildeten Ausnehmung (175) versehen ist.

14. Bandagen-System nach einem der Ansprüche 4, 12 und 13,
dadurch gekennzeichnet,
daß im Oberarmanlagebereich (OB) das Abduktionskissen (150') mit einer im Querschnitt bogenförmig verlaufenden muldenförmigen Ausnehmung (176) zur Lagenfixieirung des Oberarms in seinem Anlagebereich am Abduktionskissen (150') versehen ist.

15. Bandagen-System nach einem der Ansprüche 4, 12 bis 14,
dadurch gekennzeichnet,
daß das Abduktionskissen (150') in seiner Außenwandfläche (150'd) im Unterarmanlagebereich (UB) mit einer in Abduktionskissenlängsrichtung verlaufenden im Querschnitt bogenförmig ausgebildeten, muldenförmigen Ausnehmung (177) zur Unterarmfixierung versehen ist.

16. Bandagen-System nach einem der Ansprüche 4, 12 bis 15,
dadurch gekennzeichnet,
daß die Unterarmhalteschlingen (160, 160a, 160b) an der Außenwandfläche (150'd) des Abduktionskissens (150') aus lösbaren VELCRO-Bändern bestehen und mindestens einendseitig lösbar mit dem Abduktionskissen (150') verbunden sind.

17. Bandagen-System nach einem der Ansprüche 4, 12 bis 15,
dadurch gekennzeichnet,
daß die Unterarmhalteschlingen (160, 160a, 160b) an der Außenwandfläche (150'd) des Abduktionskissens (150') mindestens einendseitig an der Außenwandfäche (150'd) mittels eines Klettverschlusses gehalten sind.

18. Bandagen-System nach einem der Ansprüche 4, 12 bis 15,
dadurch gekennzeichnet,
daß die Halteschlaufen (160, 160a, 160b) an der Außenwandfläche (150'd) des Abduktionskissen (150') einendseitig an der Außenwandfläche (150'd) befestigt sind und mit ihrem freien Ende mittels Schnallenverschlüssen befestigbar sind.

19. Bandagen-System nach einem der Ansprüche 1 bis 18,
dadurch gekennzeichnet,
daß das Abduktionskissen (150; 150') in Längsrichtung (L) verlaufend zweigeteilt ausgebildet ist und aus zwei Formkörperteilen (F1, F2) besteht, die über oben und untenseitig verlaufende elastische Bänder (180) miteinander verbunden sind, wobei in den zwischen den beiden Formteilen (F1, F2) ausgebildeten Zwischenräume abstandveränderbare Zwischenstücke aus dem Material des Abduktionskissens (150, 150') zur Änderung des Abduktionswinkels einführbar sind.
